# EUROPEAN PATENT SPECIFICATION

(11) **EP 3 195 840 B1**
(45) Date of publication and mention of the grant of the patent: **02.06.2021**
(21) Application number: 15860539.4
(22) Date of filing: 18.09.2015
(51) Int. Cl.: A61G 1/00, A61G 1/044, A61F 5/37

(54) **DEVICE FOR IMMOBILISING INJURED CHILDREN IN A CHAIR FOR TRANSPORTING SAID CHILDREN IN VEHICLES BY ROAD, AND INSTRUCTIONS FOR USE**
VORRICHTUNG ZUR IMMOBILISIERUNG VERLETZTER KINDER IN EINEN STUHL ZUM TRANSPORT DER BESAGTEN KINDER IN FAHRZEUGEN AUF DER STRASSE SOWIE GEBRAUCHSANLEITUNG
DISPOSITIF D'IMMOBILISATION D'ENFANTS ACCIDENTÉS DANS UNE CHAISE POUR LEUR TRANSPORT DANS DES VÉHICULES ROUTIERS, ET MODE D'EMPLOI

(30) Priority: 18.11.2014 ES 201431692 P
(43) Date of publication of application: 26.07.2017
(73) Proprietor: Esquina Iglesias, Raúl, 14290 Fuente Obejuna (Cordoba) (ES)
(72) Inventor: Esquina Iglesias, Raúl, 14290 Fuente Obejuna (Cordoba) (ES)
(74) Representative: Bartrina Diaz, José María
(86) International application number: PCT/ES2015/070676
(87) International publication number: WO 2016/079350

(56) References cited:
- US-A- 4 422 454
- US-A- 4 422 454
- US-A- 4 601 075
- US-A- 4 841 961
- US-A- 4 979 520
- US-A- 5 014 374
- US-A- 5 048 134
- US-A- 5 988 173
- US-A1- 2005 245 854
- US-A1- 2006 137 097

## Description

### SUBJECT OF THE INVENTION

This invention consists of a device, according to claim 1, comprising implements and fastening straps which are intended to be placed on children in a life-threatening situation following a road accident to restrain and extract them from the car involved in the accident in order to take them to a hospital; and the bag in which such implements and fastening straps will be stored and carried.

The purpose of this invention is to provide rescue teams or health care professionals offering emergency assistance with a tool and a procedure which will allow them safely, speedily and smoothly to restrain the child involved in the accident for subsequent extraction from the vehicle and placement on a stretcher, also facilitating the ensuing handling of the patient during the medical tests to be performed in the hospital to which they will be taken, thereby reducing the risk of possible injuries being sustained or made worse during the rescue operation.

Currently, children travelling in safety car seats who have been in an accident are restrained by using fabric rolls which, once duly fitted by the rescuer, prevent the child's neck, head and torso from moving during the process of extraction from the vehicle without taking the child out the chair, as well as while removing the child from the chair and placing them on the rescue board and then on the stretcher. Preparing these rudimentary textile implements is the rescuer's job, and the use thereof requires specific training on the part of the rescuer; also, watertightness or other desirable treatments cannot be ensured insofar as such implements are made from fabric leftovers stocked up by the rescuer while preparing their work materials.

### BACKGROUND OF THE INVENTION

Different systems are currently known which perform analogous procedures, including the following:
1) ES 2 485 496 T3. This invention consists of a cushion whose purpose is to hold the child in a fixed position and a structure which uses the cushion on which the child rests for attachment to a pram seat, portable baby bed, child safety car seat or the like.
2) US-4631766. This invention consists of a baby seat especially designed to position low birth weight newborn infants with a view to preventing position-induced deformities. This baby seat effectively positions the head of a newborn infant to facilitate breathing and positions the baby's limbs in a way that prevents stress to the joints.
3) ES-2 386 237_T3. This invention is intended to absorb the impact forces exerted on the safety belt of a child car seat as a result of a sudden, big shock load, this safety belt being a vehicle seat belt which is used to hold the child safety seat in place.
4) ES-2 370 934 T3. A child protection system of this kind serves to place it in an automobile seat and comprises a back which extends a lengthwise of a longitudinal direction of the part back of backrest, with an arranged in a seat bucket to support for behind the torso of a child, and with a head support assembly disposed on the back support and movable in the longitudinal direction of the backrest, for the posterior and/or lateral support of the child's head, the bearing assembly further comprising the head of the side protective sides for the shoulders (lateral sides) which, spaced apart from each other, protrude from a lateral support section of the head support assembly.
5) ES 2 150 095 T3. Integrated child seat with automatic adaptation to the size of the child. This invention relates to vehicle-integrated child seats and, more specifically, to the way such seats are built to cover a range of child sizes.
6) ES 1 081 555 U. The subject of this model is a child retaining device which can be incorporated to, and/or used on, vehicle seats. The main characteristic of such a device is that up to four children can be anchored to a five-seat vehicle, thus making the most of all the space available while complying with the safety regulations relating to children travelling in cars.
7) ES 1 062 260 U. Cervical protection device for babies and children. This invention has been designed to protect the cervical area of babies and children travelling in cars. A problem is currently faced when babies and children travelling in cars fall asleep as, because they have no reflexes, their cervical area is most vulnerable because they cannot counteract the inertia caused every time the car is manoeuvred, even if travelling at a slow speed, such as when we take a turn onto another road or pull up at a red light, etc., all of which occur constantly within the town.
8) ES 1 058 545 U. This invention is characterized by the special configuration and design of the parts and elements of which the device is made up to create a child fastening system which allows the child to be securely fastened, even if they are on an inclined plane, while also giving them freedom to move their head and limbs, thus facilitating medical, assistance and clinical procedures.

Devices are also known (for example, those contained in US 4 422 454, US 5 014 374, US 4 979 520, US 4 601 075, US 2006/137097 and US 5 048 134) consisting of rigid brace that have been designed for placement on the patient's back and are fastened at the front with straps incorporated into the brace, immobilizing the patient's torso, or for subsequent placement on a stretcher or board with the straps fastened.

The proposed invention includes the following advantages which, therefore, constitute new features in the context of known technical solutions insofar as no references to a system meeting all the characteristics of this system are available:
- The design of this device makes the process of securing babies easier, smoother and faster.
- The device may be contracted using the user's hands, as one of the characteristics of the material is that it recovers its initial volume, adapting to the body it intercepts if this happens during such an expansion and before its full recovery (viscoelastic effect).
- The device may contain an interior rib which will give it resistance to traction without reducing the bending radius, the compression moulding capacity or the viscoelastic recovery, and it will not be an obstacle or a hindrance during the performance of subsequent medical tests, such as x-rays or other supplementary tests. The only purpose of this rib will be to provide the device with resistance to traction.
- The device includes all the elements necessary for rescue and may even include its transportation element.
- The design of its elements and the way they are arranged in the carrying container offer the advantage of favouring a procedure of use.

### EXPLANATION OF THE INVENTION

By way of an explanation of the invention, and to achieve the ends and overcome the problems described in the preceding sections, the invention consists of a specific device, according to claim 1, for immobilization of head, neck and torso of children in a life-threatening situation; as well as a set of fastening straps and a carrying and storage bag, which will prevent new injuries from being sustained, or those sustained as a result of the accident from being made worse, during rescue operations. The invention is made up of the following elements:
a. Head immobilisation device (one unit).
b. Neck immobilisation device (one unit).
c. Side torso immobilisation device (two units).
   Thus, the immobilisation devices may be built around a core, preferably textile, with high resistance to traction, total flexibility and which under no circumstance include any elements which may be visible in case of radiological tests being performed. All, some or none of the three immobilisation devices may contain such a resistant core. The main useful body is formed by a spongy body which will cover the resistant core if it does contain one. It has an elliptical cross section, it is soft and flexible and it has the capacity to deform by compression and to recover its initial shape by adapting to the outline it may intercept, if this occurs, during its expansion, without ever loosing elasticity (viscoelastic). It has a central area which may be thicker than both its ends. The dimensions -both length and diameters- will vary depending on the part of the body to be immobilised. The exterior of these elements does not have any sharp, cutting or rough edges or, generally, any shape or layout which may be aggressive to the skin of the child who has suffered the accident, as their skin will probably be scratched and will therefore require special attention and care. In addition, the device is impervious so as to prevent it from absorbing any liquids from the child who has suffered the accident, such as vomit, urine, excrements, blood or others. Similarly, treatments such as those available in hospitals involving chemical cleaning or any other kind of treatment can be applied to disinfect it. The above-described devices will also be hypoallergenic. In order to facilitate identification, it is intended that each immobilization device will be a different colour.
d) Fastening straps (three units): These consist of long strips of fabric with high mechanical resistance properties and they have a textile element for attachment (Velcro® system) which allows them to be adapted to all the dimensions of the existing baby chairs, adapting to the three possible heights, i.e. head, torso and legs (head fastening strap, torso fastening strap and leg fastening strap). As in the preceding case, these straps are impervious to prevent the absorption of any liquid from the child who has suffered the accident. They can also be given the disinfection treatments provided in hospitals and they are hypoallergenic. For ease of identification, each fastening strap is a different colour, and it is expected that such colours will correspond to the immobilisation device to be placed on the child at the same height.
e) Transportation and distribution bag arranged on the basis of the sequence of use; this consists of a bag made of a flexible material in which the aforementioned devices will be placed, stored and transported, which specifically allows these elements to be placed in an orderly manner so that they are accessed in the same order in which they are to be used. It will also have a closing flap, closure securing mechanism and handles for transportation.

The proposed invention, therefore, fully holds the child in place controlling flexion-tension and lateralisation of head and neck and immobilising the torso, so as to prevent new injuries from being sustained or those sustained during the car accident from getting worse, and it makes the child restraining process easier for the rescuer and their transportation safer.

This system also allows the child to be transported in their safety car seat, as it has the ability to adapt to the head, neck and torso, on top of the fact that the three fastening straps offer total restraint of the baby seat, thereby minimising the extent to which the child can move, whether intentionally or unintentionally, while being extracted from the vehicle or transported to the hospital.

Once the child has been extracted from the vehicle, this device can be removed and subsequently put back on once the child is on the stretcher or on the long board, or to perform a medical examination, verification of airways, application of mechanical ventilation, perform C.P.R. (Cardiopulmonary Resuscitation) or any other measure which may become necessary as a consequence of the moment and the condition of the child who has suffered the accident.

Given the device's radiolucency (transparency to X-Rays), the device can be left on the child during all the medical tests which it may be necessary to perform.

Similarly, the procedure envisaged for use of the above-described device is intended for the rescuer to hold and extract a child who, having been the victim of a road accident, needs to be extracted from the inside of the vehicle and taken to an area where proper attention can be provided to them.

Below is a sequential description of the procedure to be followed in using the device described above based on its integral elements (also described above):
a) Check that the victim's limbs are clear from any possibility of getting stuck or trapped.
b) Pre-shape the head immobilisation device with a bending radius approximately equal to the child's cranium radius. Place the device around the child's head in such a way that the child's head is centred and the device is placed between the side pillows of the child car seat on the outside and the child's head on the inside.
c) Pre-shape the neck immobilisation device with a bending radius approximately equal to the child's neck radius. Place the device around the child's neck in such a way that the child's head is centred.
d) Place each torso immobilisation device on one side of the victim's torso, trapped between the torso and the side of the child car seat. The excess length of the device may stick out from the front of the chair without any problem.
e) Finally, immobilise the child who has suffered the accident using the three fastening straps by placing each one of them in their right position, namely, securing the position of head, neck and torso and the corresponding immobilisers. No special manoeuvre is required to fasten these straps as this is done by the self-adhesive system known as Velcro®, so that:
   e.1- The first strap will be placed on the head at forehead level.
   e.2- The second strap will encompass the child's body at breastbone level (mammillary height).
   e.3- In the case of very small children, the third strap will be placed around the pelvis and, with older children, the third strap will surround the child's legs halfway down the femur.
   f) It will now be possible to take the child out of the vehicle safely in their chair. Should any of the immobilisers need to be removed, this may be done without having to remove the fastening straps and it will be similarly possible to put it back on.
   g) If necessary, all the devices can be removed in order to move the child onto a long board and, once the child is on the board, the immobilisers and the fastening straps can be put back on, thus securing the victim.

### BRIEF DESCRIPTION OF FIGURES

To complete the description offered in this document and for a better understanding of the characteristics of the invention, as per an example of recommended application, this description is accompanied by a set of figures representing, without limitation, the following:

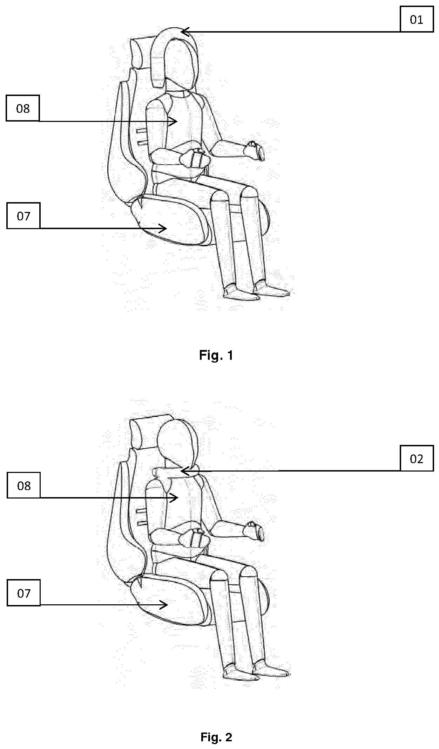

Figure 1.- Child in seat with head immobilisation device.
Figure 2.- Child in seat with neck immobilisation device.

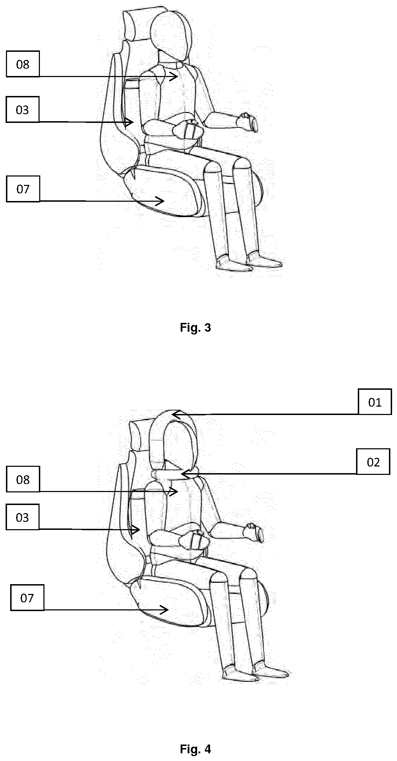

Figure 3.- Child in seat with torso immobilisation device.
Figure 4.- Child in seat with head the three immobilisation devices without the fastening straps.

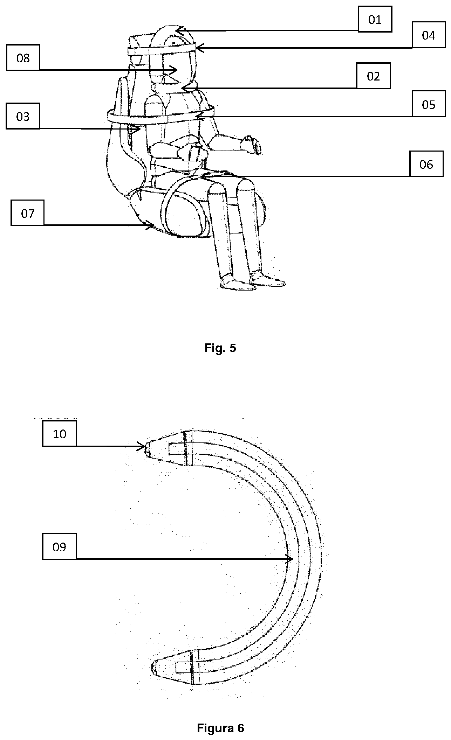

Figure 5.- Child in seat with head the three immobilisation devices with the fastening straps.
Figure 6.- Immobilisation device including resistant core.

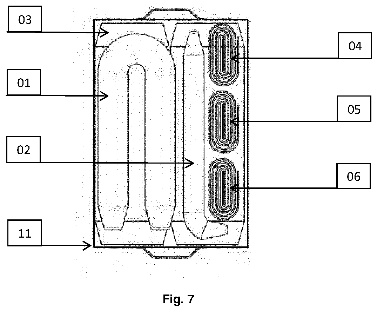

Figure 7.- Carrying bag and arrangement of devices.

Of especial note in the aforementioned figures are the following constituent parts:
01.- Head immobilisation device.
02.- Neck immobilisation device.
03.- Torso immobilisation device.
04.- Head fastening strap.
05.- Torso fastening strap.
06.- Leg fastening strap
07.- Child safety car seat.
08.- Child victim of an accident.
09.- Resistant core.
10.- Outer spongy body.
11.- Carrying bag.

### PREFERRED EMBODIMENT OF THE INVENTION

Device intended to restrain children involved in an accident for transportation in their safety car seat, which consists of the following components:
1. Head, neck and side torso immobilization devices: made up of a long, soft and flexible body with an elliptical cross section and a central area which is thicker than its two ends; this surrounds a resistant core which is highly resistant to traction and is made from a textile element or plastic-based product (e.g. nylon) to ensure invisibility to radiologic tests. By adjusting its various parts during assembly, the device as a whole - both the spine and the soft body- maintains the shape pre-configured by the user. The outer body is impervious and washable in every case, and there is a gradual reduction in diameter at the ends of every constituent part so that an approximately thirty-degree angle exists between the edges.
   1.a- Head immobilisation device (01). Red in color for ease of identification. The approximate dimensions of this element will be 8 x 4.5 x 75 [centimeters], which corresponds to ellipse diameter 1 x ellipse diameter 2 x total length. Only one unit of this element is required.
   1.b- Neck immobilisation device (02). Yellow in color for ease of identification. The approximate dimensions of this element will be 4.5 x 3 x 38 [centimeters], which corresponds to ellipse diameter 1 x ellipse diameter 2 x total length. Only one unit of this element is required.
   1.c- Torso immobilisation device (03). Green in color for ease of identification. The approximate dimensions of this element will be 11 x 8 x 33 [centimeters], which corresponds to ellipse diameter 1 x ellipse diameter 2 x total length. Two units of this element are required, one for each side of the torso.
2. Fastening straps. Straps made from a soft-to-the-touch material, without any rigid, cutting or rough parts so as to prevent damage to the victim or discomfort. They are resistant to traction, with virtually no elasticity; they are flexible to torsion and bending and have various textile elements of the Velcro® type which allow each strap to be quickly fastened to their working position without pressing and without button fittings or hooks or any other system which would require skill and special attention on the part of the rescuer. The fastening straps to be provided are: head fastening strap (04), torso fastening strap (05) and leg fastening strap (06), so that the head fastening strap will be placed on the child's head at forehead level, the torso fastening strap will surround the child's body at breastbone level (mammillary height) and, with very small children, the leg fastening strap will be placed around the pelvis while, with older children, the third strap will surround the child's legs halfway down the femur.
   Similarly, the dimensions and the positioning, in various stretches, of the rope fitting system, will enable adjustment to all child safety car seat models, as well as fixation to a board, stretcher or any other element commonly used by healthcare professionals or rescue teams. All these fastening straps will measure 150 x 3.5 [centimeters], which corresponds to length by width. Therefore, there will be three fastening straps of equal dimensions and constructive characteristics but different in colour so as to identify their positions, each coinciding with its corresponding immobiliser -i.e. red, yellow and green.
3. Carrying and distribution bag for arrangement of elements in sequence of use. It consists of a fabric structure with side handles and an easy-to-open and easy-to-close flap, as well as shoulder straps for easy carrying. Inside this bag there is a storage housing to identify the correct position of each element whose colour makes it differentiable from the items it carries and holds, such as a dark ochre colour. The above-described elements will be arranged as follows: first, on the base of the packing, the two torso immobilisers (03) will be laid out in a fully stretched position. On top of these will be, on one side, the head immobiliser (01) in a U-shaped position so that it can be correctly immobilised, and, on the other side, the neck immobilisation device (02) fully stretched and the three fastening straps (head fastening strap (04), torso fastening strap (05) and leg fastening strap (06)). Thus, the approximate dimensions of the carrying bag will be 34x23x17 [centimetres], which corresponds to length, depth and height respectively.

## Claims

1. Device for immobilising injured children in a chair for transporting said children in vehicles by road, wherein said device comprises:
one head immobilisation device (1);
one neck immobilisation device (2);
two torso immobilisation devices (3);
a head fastening strap (4);
a torso fastening strap (5);
a leg fastening strap (6);
a carrying and distribution bag (11) for arrangement of elements in sequence of use;
wherein the head immobilisation device (1), the neck immobilisation device (2) and the torso immobilisation devices (3) are built around a core (9), preferably textile, with high resistance to traction, total flexibility, and do not include any elements which are visible in case of radiological tests; these immobilization devices have a spongy body (10) with an elliptical cross section which is formed by a long, soft and flexible body, with capacity to deform by compression and to recover its initial shape by adapting to the outline it may intercept, if this occurs, during its expansion, without ever loosing elasticity, which will cover the core (9); this spongy body (10) has a gradual reduction in diameter at the ends of every constituent part so that an approximately thirty-degree angle exists between the edges, having a central area which is thicker than both its ends; the outer body of the head immobilisation device (1), the neck immobilisation device (2) and the torso immobilisation devices (3) is impervious to liquids and washable; in order to facilitate identification, each immobilization device is a different colour;
and the head (4), torso (5) and leg (6) fastening straps consist of long strips of fabric with high mechanical resistance properties and having a textile element for attachment; these fastening straps are also impervious to prevent the absorption of any liquid from the child who has suffered the accident, and each fastening strap is a different colour; and the carrying and distribution bag (11) for the arrangement of elements in sequence of use is made of a flexible material and also has a closing flap, closure securing mechanism and handles for transportation.

## Patentansprüche

1. Vorrichtung zum Immobilisieren verletzter Kinder in einem Stuhl zum Transportieren der Kinder in Fahrzeugen auf der Straße, wobei die Vorrichtungen umfassen:
eine Kopfimmobilisierungsvorrichtung (1);
eine Halsimmobilisierungsvorrichtung (2);
zwei Vorrichtungen zur Immobilisierung des Rumpfes (3);
einen Kopfbefestigungsgurt (4);
einen Rumpfbefestigungsgurt (5);
einen Beinbefestigungsgurt (6);
ein Trage- und Verteilerbeutel (11) zur Anordnung der Elemente in der Reihenfolge ihrer Verwendung;
wobei
die Kopfimmobilisierungsvorrichtung (1), die Halsimmobilisierungsvorrichtung (2) und die Rumpfimmobilisierungsvorrichtung (3) um einen Kern (9), vorzugsweise textil, mit hoher Zugfestigkeit, vollständiger Flexibilität und ohne bei radiologischen Tests sichtbare Elemente aufgebaut sind; diese Immobilisierungsvorrichtungen haben einen schwammigen Körper (10) mit einem elliptischen Querschnitt, der von einem langen, weichen und flexiblen Körper gebildet wird, der sich durch Kompression verformen und seine ursprüngliche Form durch Anpassung an den Umriss wiederherstellen kann, den er abfangen kann, wenn dies findet während seiner Expansion statt, ohne jemals die Elastizität zu verlieren, die den Kern (9) bedeckt; dieser schwammige Körper (10) hat an den Enden jedes Bestandteils eine allmähliche Verringerung des Durchmessers, so dass zwischen den Kanten ein Winkel von ungefähr dreißig Grad besteht, der einen zentralen Bereich aufweist, der dicker als beide Enden ist; der äußere Körper der Kopfimmobilisierungsvorrichtung (1), der Halsimmobilisierungsvorrichtung (2) und der Vorrichtungen zur Immobilisierung des Rumpfes (3) ist flüssigkeitsundurchlässig und waschbar; um die Identifizierung zu erleichtern, hat jede Immobilisierungsvorrichtung eine andere Farbe; und die Befestigungsgurte für Kopf (4), Rumpf (5) und Bein (6) bestehen aus langen Gewebestreifen mit hohen mechanischen Beständigkeitseigenschaften und einem textilen Element zur Befestigung; diese Befestigungsgurte sind auch undurchlässig, um die Aufnahme von Flüssigkeit von dem Kind zu verhindern, das den Unfall erlitten hat, und jeder Befestigungsgurt hat eine andere Farbe; und wobei der Trage- und Verteilerbeutel (11) zur Anordnung von Elementen in der Reihenfolge der Verwendung aus einem flexiblen Material besteht und auch eine Verschlussklappe, einen Verschlusssicherungsmechanismus und Griffe für den Transport aufweist.

## Revendications

1. Dispositif pour l'immobilisation des enfants blessés dans une chaise pour le transport desdits enfants dans des véhicules par route, dans lequel lesdits dispositifs comprennent:
un dispositif d'immobilisation de la tête (1);
un dispositif d'immobilisation du cou (2);
deux dispositifs d'immobilisation du torse (3);
une sangle de fixation de la tête (4);
une sangle de fixation du torse (5);
une sangle de fixation de la jambe (6);
un sac de transport et de distribution (11) pour l'agencement des éléments dans la séquence d'utilisation;
où
le dispositif d'immobilisation de la tête (1), le dispositif d'immobilisation du cou (2) et les dispositifs d'immobilisation du torse (3) sont construits autour d'un noyau (9), de préférence textile, avec une haute résistance à la traction, une flexibilité totale, et ne pas comprennent tous les éléments qui sont visibles dans le cas d'examens radiologiques; ces dispositifs d'immobilisation ont un corps spongieux (10) avec une section transversale elliptique qui est formée par un corps allongé, souple et flexible, avec une capacité à se déformer par compression et à reprendre sa forme initiale en l'adaptant au contour qu'il peut intercepter, si cela se produit, lors de son expansion, sans jamais perdre d'élasticité, ce qui recouvrira le noyau (9); le corps spongieux (10) présente une diminution progressive du diamètre au niveau des extrémités de chaque partie constitutive de telle sorte qu'un angle d'environ trente degrés existe entre les bords, ayant une zone centrale qui est plus épaisse que ses deux extrémités; le corps externe du dispositif d'immobilisation de la tête (1), le dispositif d'immobilisation du cou (2) et les dispositifs d'inmobilisation du torse (3) est imperméable aux liquides et lavable; afin de faciliter l'identification, chaque dispositif d'immobilisation a une couleur différente; et les sangles de fixation de la tête (4), du torse (5) et de la jambe (6) consistent en de longues bandes de tissu ayant des propriétés élevées de résistance mécanique et ayant un élément textile pour la fixation; ces sangles de fixation sont également imperméables pour empêcher l'absorption d'un liquide à partir de l'enfant qui a subi l'accident, et chaque sangle de fixation est d'une couleur différente; et le sac de transport et de distribution (11) pour l'agencement des éléments dans la séquence d'utilisation est fabriqué d'un matériau souple et possède également un rabat de fermeture, un mécanisme de fixation pour la fermeture et des poignées pour le transport.
